(19) **Europäisches Patentamt European Patent Office Office européen des brevets**

(11) **EP 2 358 265 B1**

(12) **EUROPEAN PATENT SPECIFICATION**

(45) Date of publication and mention
of the grant of the patent:
**02.01.2013 Bulletin 2013/01**

(51) Int Cl.:
***A61B 5/00*** (2006.01)

(21) Application number: **09786426.8**

(22) Date of filing: **15.06.2009**

(86) International application number:
**PCT/IB2009/052539**

(87) International publication number:
**WO 2010/058302 (27.05.2010 Gazette 2010/21)**

(54) **NEEDLE WITH INTEGRATED FIBERS**

NADEL MIT INTEGRIERTEN FASERN

AIGUILLE AVEC FIBRES INTÉGRÉES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **19.11.2008 EP 08169427**

(43) Date of publication of application:
**24.08.2011 Bulletin 2011/34**

(73) Proprietor: **Koninklijke Philips Electronics N.V.**
**5621 BA Eindhoven (NL)**

(72) Inventors:
• **BRAUN, Augustinus, L.**
**NL-5656 AE Eindhoven (NL)**
• **HENDRIKS, Bernardus, H., W.**
**NL-5656 AE Eindhoven (NL)**
• **HARBERS, Rik**
**NL-5656 AE Eindhoven (NL)**
• **VAN DER VOORT, Marjolein**
**NL-5656 AE Eindhoven (NL)**
• **DESJARDINS, Adrien, E.**
**NL-5656 AE Eindhoven (NL)**
• **NACHABE, Rami**
**NL-5656 AE Eindhoven (NL)**
• **WASSINK, Bernardus, W., J.**
**NL-5656 AE Eindhoven (NL)**
• **VAN GAAL, Franciscus, M., A., M.**
**NL-5656 AE Eindhoven (NL)**
• **MIHAJLOVIC, Nenad**
**NL-5656 AE Eindhoven (NL)**
• **BABIC, Drazenko**
**NL-5656 AE Eindhoven (NL)**

(74) Representative: **Kroeze, Johannes Antonius**
**Philips**
**Intellectual Property & Standards**
**P.O. Box 220**
**5600 AE Eindhoven (NL)**

(56) References cited:
**WO-A1-01/78596**      **WO-A1-91/06271**
**WO-A2-2008/008318**   **US-A- 5 460 182**
**US-A- 5 674 184**     **US-A1- 2005 043 589**

**Description**

FIELD OF THE INVENTION

The invention generally relates to a needle with integrated fibers. Particularly, the invention relates to a small diameter needle for tissue inspection based on optical spectroscopy to diagnose whether tissue is cancerous or not.

TECHNOLOGICAL BACKGROUND

Needle interventions are widely used in the field of oncology for taking biopsies of tissue in order to inspect whether tissue is cancerous or not. To make these interventions more reliable feedback of what kind of tissue is in front of the needle is required. A way to achieve this is by making use of optical spectroscopy. This requires integration of fibers into the needle. These fibers are used to deliver light to illuminate the tissue in front of the needle and to collect back the reflected light from the tissue.

However, a problem with this is how to integrate these fibers into the needle without compromising the original functionality of the needle, hence altering the outer part of the needle or the inner part (for instance used to take a tissue sample). This means that the fibers must be integrated inside the needle wall while still being able to construct different fibers arrangements at the distal end of the needle. This is of particular importance, because the fiber arrangement at the tip of the needle determines the sensitivity in the measurement of certain optical properties. For instance the fact that a fiber tip is slanted or the distance between two fibers at the tip, affects the measured signal.

Therefore, needles employing optical fibers are particular suited to provide physiological information of the tissue in front of the needle. Such a needle containing fibers require that these fibers are connected to an optical console. At the console light may be coupled into these fibers to illuminate the tissue in front of the needle, while light, being backscattered from the tissue and coupled back into the fiber, may be detected in the console and further processed. This means that the needle may be connected to the conso le by the fiber.

This construction has several drawbacks: First of all, the connector fiber part can easily break or being damaged during handling. Furthermore, the connector fiber part makes the sterilization of the needle more difficult hence adding extra costs. Finally, handling by the doctor when setting up the needle for usage is cumbersome because of usually long fibers.

A needle according to the preamble of claim 1 is known from WO 2008/008318.

SUMMARY OF THE INVENTION

It is an object of the invention to manufacture a needle having at least one fiber exit at the distal end of the needle, where the fiber does not obstruct the hollow part of the needle if present as well as does not extend beyond the outer cylinder geometry. It is another object of the invention, to provide a needle which can be used reliably, which will be easily handled, and which can be appropriately sterilized.

These objects are achieved by the subject matter according to each of the independent claims. Further embodiments of the present invention are described in the respective dependent claims.

Generally, it is proposed to manufacture a needle consisting of five parts: an inner cylinder tube, an outer hollow cylinder tube, a needle tip part with integrated fiber exit, a connector part, and a holder part.

A needle according to the invention comprises a tip part comprising a through bore in axial direction, a holder part comprising an opening, a shaft comprising an inner tube and an outer tube, wherein distal ends of the inner and outer tubes are connected with the tip part, and proximal ends of the inner and outer tubes are connected with the holder part, and wherein a space is formed between the inner tube and the outer tube, a fiber, the fiber being capable of transmitting light, wherein an end section of the fiber is located in the through bore of the tip part, wherein the fiber is located in the space formed by the inner and outer tubes of the shaft, and wherein the fiber passes through the opening of the holder part.

It should be noted that the hollow spacing between the inner and the outer cylinder is larger than or equal to the outer diameter of the fibers, the inner and the outer cylinder are mounted in the holder part, and the tip part is mounted on the two cylinders.

Advantageously, the use of the two cylinders having different diameters enables an easy way to incorporate the fiber into the needle. Furthermore, the use of a separate tip part enables a large freedom in assembling the illumination and collection fiber end geometries without having to make a complex mount that extends over the entire needle length.

The holder part is provided with a channel to guide the fibers from the tip part, being mounted in between the two cylinders, towards the outside world. This part has as additional functionality to provide strength around the fiber to prevent large bending angles of the fibers at the proximal end of the needle.

The cylinders and the needle tip might be made of metal, wherein the metal might be MRI compatible such as Titanium. The needle tip might also be made of a ceramic material. This has the advantage of being mouldable in various shapes while still allowing for a sharp and robust needle tip. Further, the holder part might be made by plastic injection moulding.

According to one embodiment of the invention, the tip part of the needle further comprises a shaft portion and a bevel forming the tip of the needle, wherein the fiber might be located in the through bore such that the end surface of the fiber is flush with the surface of the bevel.

In case of two fibers, wherein both fibers being capable of transmitting light, an end surface of one of the fibers might be located at a top of the bevel and the other one of the fibers might be located at a bottom of the bevel.

The bevel of the needle is in general slanted in order to allow easy entry into the tissue. Therefore, with 'bevel' is meant a geometrical structure allowing for introducing the needle into tissue. Usually, a shaft of a needle includes a circular cross section. The distal end of a needle shaft, in particular of a shaft of a hollow needle, is cut such that an oval surface is formed, which is inclined relative to the longitudinal axis of the shaft. Further, there is defined an angle between the longitudinal axis of the shaft and the inclined surface, i.e. the bevel. The bevel forms a pointed tip at the most distal end of the needle. Furthermore, the edge between the outer surface of the shaft and the inclined surface of the bevel might be sharpened.

The wording 'top of the bevel' should indicate an area being part of the surface of the bevel, which area is located adjacent to the distal edge between the bevel and the shaft. That is, a fiber which is located at the top of the bevel might be located at the long axis of the oval surface of the bevel, near the distal edge, i.e. the pointed tip.

On the other hand, 'bottom of the bevel' means the area being part of the surface of the bevel, which area is located diametric to the top of the bevel. That is, the fiber which is located at the bottom of the bevel might be on or near or adjacent beside the long axis of the oval surface of the bevel near the proximal edge between bevel and shaft.

However, the wording 'bevel' might also include similar structures at the tip of the needle, which structures are useful for introducing the needle into a tissue. For example, the bevel might be a convex or concave surface, or the bevel might be a combination of several small surfaces, wherein these surfaces are connected to each other by steps or edges. It might also be possible that the cross section of the shaft is not completely cut by the bevel, such that an area remains which is blunt, i.e. is perpendicularly orientated relative to the longitudinal axis of the shaft. Such a 'blunt' end might include rounded edges or might also form a rounded leading edge. As another exemple, a sharp edge might be formed by two or more slanted surfaces being symmetrically or asymmetrically arranged to form the tip of the needle.

It should be noted that the bevel might form an acute angle with the shaft, such that the needle includes a pointed tip. Preferably, the acute angle might be approximately 20°.

According to one embodiment of the invention, the shaft of the needle has an outer diameter, and the end surfaces of the fibers are arranged at a distance to each other, wherein the distance between the end surfaces is greater than the diameter. Preferably, the distance between the fiber ends is greater than the diameter of the shaft. For example, the distance is more than 1.1 times greater than the diameter. Particularly, the distance is more than 1.25 times greater than the diameter. Preferably, the distance is more than 1.5 times greater than the diameter.

Depending on the intended use of the needle, the outer diameter of the needle might be 2.108 mm for a brain biopsy needle, between 1.27 mm and 2.108 mm for a common biopsy needle or a neuro puncture needle, between 0.711 mm and 2.108 mm for a fine aspiration needle, between 0.711 mm and 1.473 mm for an epidural needle, and might be 2.108 mm or smaller for a needle electrode.

According to a further embodiment of the invention, the needle further comprises a third fiber which is capable of transmitting light, wherein an end surface of the third fiber is located at the bottom of the bevel in the vicinity of the end surface of the other one of the fibers. In this case, the other one of the fiber and the third fiber might be located beside the long axis of the bevel surface.

For example, with a needle diameter of 1.3 mm it might be possible that the distance between the fiber at the top of the bevel and one of the fibers at the bottom of the bevel might be 2.46 mm, and the distance between the two fibers at the bottom of the bevel might be 0.37 mm.

It is noted that the distances are measured from the central axis of one of the fibers to the central axis of the other one of the fibers.

According to yet another embodiment of the invention, the needle further comprises a connector part, wherein a proximal end section of the fiber is located in the connector part for connection with a fiber cable located between the needle and a console including a light source and a light detector. With this, the proximal fiber end is rigidly mounted on the needle. In other words, there will be no fiber section leading out of the holder part of the needle.

Advantageously, the manufacturing, sterilization and handling of the needle is significantly simplified, the separate fiber cable can be made more robust since this part may be reused, and the workflow for setting up the equipment becomes easier since this separate fiber cable can be setup beforehand, i.e. the needle need not to be unpacked from its sterile enviroment.

To produce a needle according to the invention, it is proposed that the corresponding method generally comprises the steps of manufacturing the tip part including forming at least one through bore in axial direction, positioning and fixing an end section of at least one fiber in a respective through bore, connecting distal ends of the inner tube and the outer tube with the tip part, such that the at least one fiber is located in a space formed between the inner and outer tubes, passing the at least one fiber through an opening of the holder part, and connecting proximal ends of the inner tube and the outer tube with the holder part.

The method might further comprise the step of polishing the surface of the bevel and the end surface of the at least one fiber such that the end surface of the at least one fiber is flush with the surface of the bevel.

According to an embodiment of the invention, the fixing of the at least one fiber in the respective through bore might be provided by gluing, and the connecting of the inner and outer tubes with at least one of the tip part and the holder part, might be provided by welding or by gluing.

According to a further embodiment of the invention, the method may further comprise the step of fixing a proximal end of the at least one fiber in a connector part located at the holder part, so that the fiber is rigidly mounted in the fiber.

According to another embodiment of the invention, the needle with fibers might be used in a system for optical tissue inspection, wherein the system further comprises a light source connected with one of the fibers of the needle, a light detector connected with another one of the fibers of the needle, wherein light coming from the light source and being emitted from the end surface of the one of the fibers can be detected by the light detector when entering the other one of the fibers, a processing unit for processing the data from the light detector, and a monitor for visualization of the processed data. Between the needle and the light source and/or the light detector, a separate fiber cable may be provided.

In such a system, the fiber distal ends in the needle slanted bevel, as mentioned above, provide at least one source-detector fiber pair with a distance A that is larger than the outer diameter of the needle D, wherein A > 1.1D or even A > 1.25D, and preferably A>1.5D. If b is the tip angle of the needle bevel the following equation might count

$$\frac{A}{D} > \frac{\sin b + 0.1}{\sin b} \qquad (1)$$

In the case that the needle is provided with a fiber at the top of the bevel, and with two fibers at the bottom of the bevel, the fiber at the top might serve as a light source emitting light into surrounding tissue, and the two other fibers might be two detector fibers collecting reflected light. It should be noted, that other cominations are also possible regarding attaching the fibers to the light source and detector, respectively.

The invention might also be related to a computer program for the processing unit of the system according to the invention. The computer program is preferably loaded into a working memory of a data processor. However, the computer program may also be presented over a network like the worldwide web and can be downloaded into the working memory of a data processor from such a network. The computer program might control the emitting of light, might process the signals coming from the light detector at the proximal end of the detector fiber(s). These data might then be visualized at a monitor.

It has to be noted that embodiments of the invention are described with reference to different subject matters. In particular, some embodiments are described with reference to method steps whereas other embodiments are described with reference to devices or systems. However, a person skilled in the art will gather from the above and the following description that, unless other notified, in addition to any combination of features belonging to one type of subject matter also any combination between features relating to different subject matters is considered to be disclosed with this application.

The aspects defined above and further aspects, features and advantages of the present invention can also be derived from the examples of embodiments to be described hereinafter and are explained with reference to examples of embodiments. The invention will be described in more detail hereinafter with reference to examples of embodiments but to which the invention is not limited.

BRIEF DESCRIPTION OF THE DRAWINGS

Figure 1 shows a side view and a top view of a needle according to one embodiment of the invention.
Figure 2 shows an isometric view of the tip part of a needle according to an embodiment of the invention.
Figure 3 shows a system according to the invention, including a needle according to another embodiment of the invention.
Figure 4 illustrates in a schematic over view and in a detailed view, the aspect of connecting a separate fiber cable with the needle according to the invention.

Figure 5 shows a flow chart illustrating different steps of a method for producing a needle according to the invention.

The illustration in the drawings is schematically only and not to scale. It is noted in different figures, similar elements are provided with the same reference signs.

DETAILED DESCRIPTION OF EXEMPLARY EMBODIMENTS

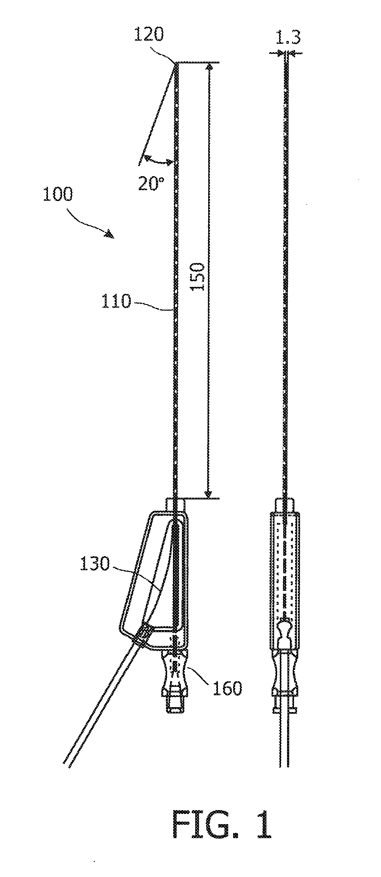

As illustrated in figure 1, a needle 100 according to one embodiment of the invention, comprises a shaft 110, a bevel 120 at the tip portion of the shaft, a fiber 130, and a holder part 160. According to this exemplary embodiment, the shaft has a length of 150 mm and a diameter of 1.3 mm. Further, the bevel encloses an angle with the shaft axis of 20°.

In this embodiment, the fiber 130 which runs from the distal end, i.e. the surface of the bevel 120, through the shaft 110 to the holder part 160, passes through an opening of the holder part 160 out of the needle.

It is noted that the dimensions mentioned above, are exemplary and not intended to limit the scope of the invention. With the mentioned dimensions, it is intended to provide an order of magnitude and relations for a needle for tissue inspection based on optical spectroscopy.

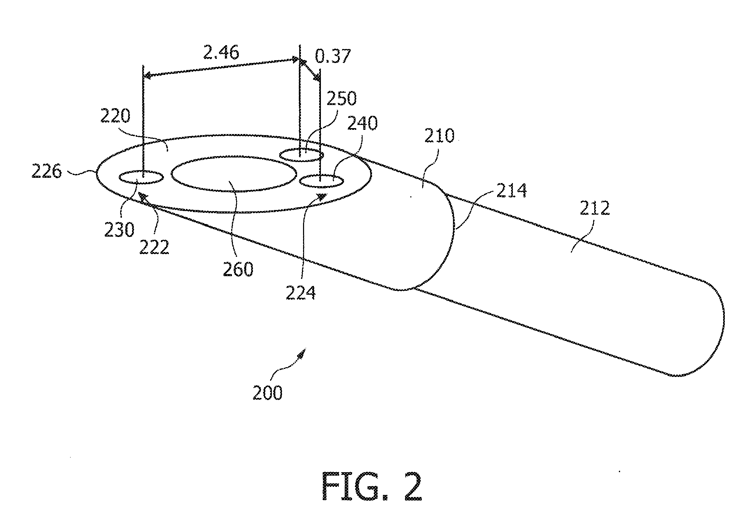

In figure 2, there is illustrated a tip part of a needle according to an embodiment of the invention. This tip part is formed before the needle will be assembled. The tip part might be made of an appropriate metal material or alloy or might, preferably, be made of a ceramic material.

The tip part 200 includes a shaft portion having a thicker section 210 and a thinner section 212. Between said shaft sections, a step or shoulder 214 is formed. The tip part further comprises a bevel 220 with a top 222 and a bottom 224, wherein the top is a surface area near the pointed tip 226 of the tip part 200. Parallel to the longitudinal axis of the shaft portion, there are provided three small through bores or channels 230, 240 and 250. Each of said small through bores is formed such that an opening of each bore is at the bevel surface 220 and the other opening of the bore is in the surface of the shoulder 214, which surface is orientated substantially perpendicular to the longitudinal axis of the shaft portion. The bores 230, 240 and 250 are dimensioned so that a fiber might fit into that bore, wherein the fiber might additionally be fixed by gluing.

As depict in figure 2, just exemplary and not intended as restriction, the distances between the bores are enclosed, measured from the centre of the respective bore. Here, the distance between the bore 230 at the top 222 of the bevel 220 and the bore 250 at the bottom 224 of the bevel 220 is 2.46 mm. The distance between the two bores 240, 250 at the bottom 224 of the bevel 220 is 0.37 mm.

Finally, the tip part 200 includes a channel 260 along the centre axis of the shaft portion. Such a channel 260 might serve to deliver, for example, drugs or to extract substances from the tissue in which the needle is positioned.

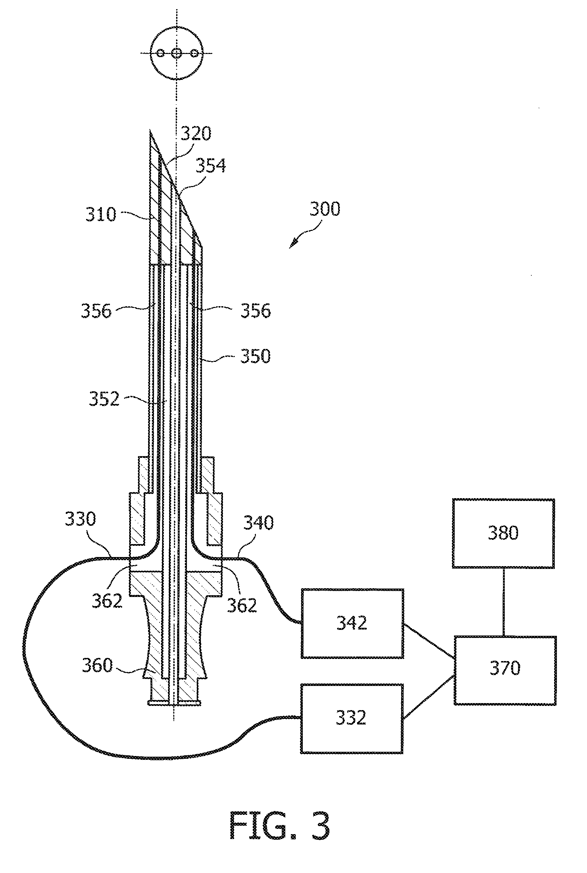

Figure 3 illustrates a system according to the invention. The system includes a needle 300 according to an embodiment of the invention. In this illustration, the needle 300 is an assembly of a tip part 310, an inner tube 352, an outer tube 350, and a holder part 360. Furthermore, two fibers 330 and 340 are shown in the needle.

An important part of the needle is the needle tip (see figure 2), in which two or three bores are manufactured. In each bore a fiber is mounted, by gluing. The tip is fixed to both inner tube and outer tube by welding or gluing, wherein the inner and outer diameters of the inner and the outer tube are adapted to corresponding diameters of the thicker and the thinner shaft section of the tip part. A space 356 between the tubes might be achieved, into which the through bores in the tip part are open out. Coming out of the bores in the tip part, the fibers 330, 340 are positioned in the hollow space 356 between both tubes.

The tip, fibers and both tubes, once assembled, is fixed to a needle holder. Inside the holder the inner tube is connected with a connector to which for instance a syringe or other tubing can be fixed. In this way volumes of fluid can be dispensed through the channel 354 of the inner tube and tip part, without interaction with the fibers. The needle holder 360 also contains separate exit 362 for the fibers. After assembling tip, fibers, tubes and holder, the bevel 320 of the needle (i.e. the needle tip) is polished to obtain a proper surface quality for the fibers.

Further, the system comprises a light source 332, a light detector 242, a processing unit 370 and a monitor 380. The processing unit 370 is capable of controlling the light source 332 to emit light into the fiber 330 such that light will be emitted through the distal end surface of the fiber 330 at the top of the bevel 320 into surrounding tissue. Depending on what kind of tissue is in front of the bevel, more or less of the emitted light will be reflected in the direction of the bottom of the bevel, to be received be the other fiber 340. Through the fiber 340, the light will be led to the light detector 342, which detector is adapted to transform the light into electrical signals. These electrical signals will be send by, for example, wire to the processing unit. The processing unit will process the data corresponding to the electrical signals, so that the processed data might be visualized on a monitor 380. Based on said visualized data, it might be possible to diagnose whether or not a tissue is cancerous.

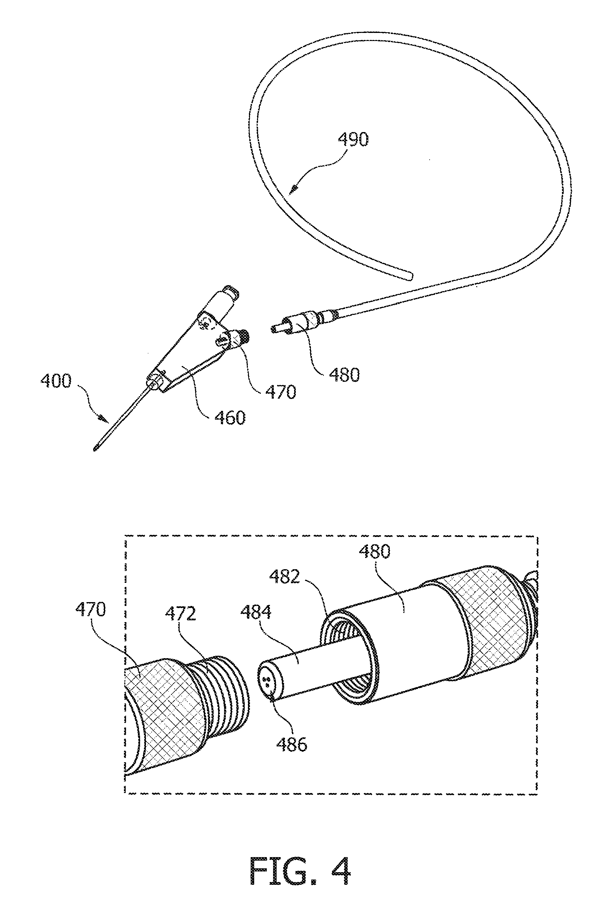

Figure 4 illustrates a connection of a fiber cable 490 with the holder part 460 of the needle 400. To make the connection to a console, a separate fiber connector cable 490 may be used or a fiber cable that is pre-connected to the console. This separate fiber cable need not be sterilized. In case this is required a plastic sterile sleeve can be put around it as is already commonly used in medical equipment.

To couple the needle 400 with the fiber cable 490, there is provided a connector part 470 at the needle 400, and a correspondingly formed connector part 480 at the fiber cable 490. As depicted in the detail view in figure 4, the connector part 470 which is mounted at the holder part 460, includes a smaller end portion 472 and a recess (not shown) in its front surface. On the other hand, the connector part 480 at the fiber cable 490 includes in axial direction, a hollow portion 482, a pin like element 484 and a front surface 486.

During connection of the connector parts 470, 480, the pin like element 484 will be accommodated in the recess of the connector part 470, and the smaller end portion 472 will engage in the hollow portion 482 of the counter connector part 480. Flush with the blunt front surface 486 of the connector part 480, there is positioned at least one fiber end. The corresponding end(s) of the fiber(s) in the needle are located in the end surface of the recess of the connector part 470. Therefore, an appropriate and reliable connection between the two connector parts and thus between the fibers of the needle and the fiber cable is easily realized.

It is noted that a rigid connector 470 on the needle can either connect one fiber in the needle or a multiple number. In figure 4, there are three fiber ends visible at the end surface 486 of the pin like element 484 of the connector part 480 at the end of the fiber cable 490.

The construction according to the invention has the following advantages, namely that manufacturing, sterilization and handling of the needle is significantly simplified, that the separate fiber cable can be made more robust since this part is being reused, and that the workflow for setting up the equipment becomes easier since this separate fiber cable can be setup beforehand, i.e. the needle need not to be unpacked from its sterile environment.

Beside that there is only one connector present on the needle, also multiple connectors are envisioned. The connector may also provide connection to other signals such as electrical signals.

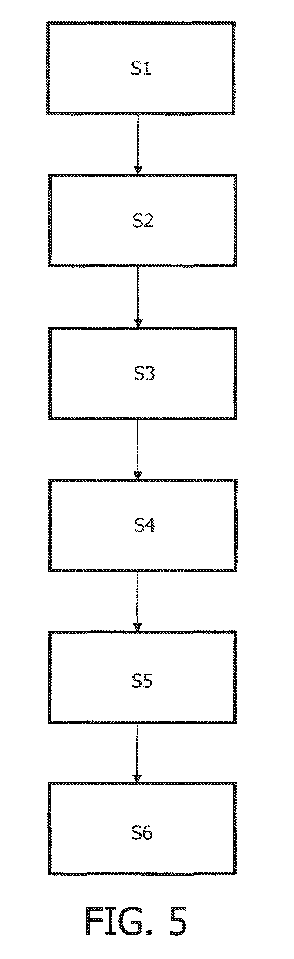

Figure 5 is a flow chart, showing the steps of a method for producing a needle according to the invention. It will be understood, that the steps described with respect to the method, are major steps, wherein these major steps might be differentiated or divided into several sub steps. Furthermore, there might be also sub steps between these major steps. Therefore, a sub step is only mentioned, if said step is important for the understanding of the principles of the method according to the invention.

Step S 1 of the method according to the invention, is the manufacturing of the tip part, wherein this manufacturing includes forming of at least one through bore in axial direction.

Step S2 is the positioning of an end section of at least one fiber in a respective through bore, wherein said positioning might include the fixing of the at least one fiber by, for example, gluing.

Step S3 is the connecting of the distal ends of the inner tube and the outer tube with the tip part, such that the at least one fiber is located in a space formed between the inner and outer tubes.

Step S4 is the connecting of the proximal ends of the inner tube and the outer tube with the holder part, wherein the at least one fiber will be passed through an opening of the holder part.

Step S5 is the fixating of the proximal end of the at least one fiber by means of a connector part mounted at the holder part.

Step S6 of the method according to the invention, is the polishing of the surface of the bevel and of the end surface of the at least one fiber such that the end surface of the at least one fiber is flush with the surface of the bevel.

In the following, exemplary needles according to the invention will be described with respect to their outer diameter, their insertion length, and their preferred use.

A biopsy needle might have an outer diameter of 1.27 mm up to 2.108 mm, might be inserted into tissue with 100 mm to 150 mm of its length, and might be used in soft tissue core biopsies in the neck, the head, the breast, the prostate, and the liver.

A fine aspiration needle of soft tissue might have an outer diameter between 0.711 mm and 2.108 mm, might be inserted into soft tissue with 100 mm to 150 mm of its length, and might be used for aspiration of soft tissue.

A brain biopsy needle might have an outer diameter of 2.108 mm, might be inserted into tissue with 150 mm up to 250 mm of its length, and might be used for diagnostic brain biopsies.

A neuro puncture needle might have an outer diameter of 1.27 mm up to 2.108 mm, might be inserted into tissue with 150 mm to 200 mm of its length, wherein such needles allow a non-traumatic approach to lesions in the brain.

An epidural needle might have an outer diameter between 0.711 mm and 1.473 mm, might be inserted into tissue with a length of up to 150 mm, and might be used for treatments in the spinal cord area such as steroid injections in the epidural space.

Finally, a needle electrode might have an outer diameter of 2.108 mm and smaller, might be inserted into tissue up to 250 mm of its length, and might be used for radiofrequency ablation for instance of tumors.

While the invention has been illustrated and described in detail in the drawings and foregoing description, such illustration and description are to be considered illustrative or exemplary and not restrictive; the invention is not limited to the disclosed embodiments. The scope of the invention is defined in the claims. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE SIGNS

| | |
|---|---|
| 100, 300, 400 | needle |
| 110 | shaft |
| 120, 220, 320 | bevel |
| 210 | thicker section of shaft portion |
| 212 | thinner section of shaft portion |
| 214 | sho lder |
| 222 | top of the bevel |
| 224 | bottom of the bevel |
| 130, 230, 240, 250, | fiber |
| 200, 310 | tip part |
| 330,340 | fiber |
| 332 | light source |
| 342 | light detector |
| 350 | outer tube |
| 352 | inner tube |
| 260, 354 | channel |
| 356 | space between inner and outer tubes |
| 160,360,460 | holder part |
| 362 | opening |
| 370 | processing unit |
| 380 | monitor |
| 470, 480 | connector part |
| 472 | smaller end portion |
| 482 | hollow portion |
| 484 | pin like element |
| 486 | front surface |
| 490 | fiber cable |

**Claims**

1.  A needle (100, 300, 400) comprising:

    - a tip part (200, 310) comprising a through bore in axial direction,
    - a holder part (160, 360, 460) comprising an opening,
    - a shaft (110) comprising an inner tube (352) and an outer tube (350), wherein distal ends of the inner and outer tubes are connected with the tip part (200, 310), and wherein a space (356) is formed between the inner tube and the outer tube,
    - a fiber (130, 230, 240, 250, 330, 340), the fiber being capable of transmitting light, wherein an end section of the fiber is located in the through bore of the tip part (200, 310), and wherein the fiber passes through the opening of the holder part, **characterised in that**:

        the proximal ends of the inner and outer tubes are connected with the holder part (160, 360, 460), and the fiber is located in the space (356) formed by the inner and outer tubes of the shaft (110).

2. The needle of claim 1, wherein the tip part (200, 310) further comprises a shaft portion and a bevel (120, 220, 320) forming the tip of the needle.

3. The needle of claim 1, wherein the fiber (130, 230, 240, 250, 330, 340) is located in the through bore such that the end surface of the fiber is flush with the surface of the bevel (120, 220, 320).

4. The needle of claim 2, wherein an end surface of the fiber (130, 230, 330) is located at a top (222) of the bevel (120, 220, 320).

5. The needle of claim 1, comprising two fibers (130, 230, 240, 330, 340), both fibers being capable of transmitting light, wherein an end surface of one of the fibers (130, 230, 330) is located at a top (222) of the bevel (120, 220, 320) and the other one of the fibers (240, 340) is located at a bottom (224) of the bevel.

6. The needle of claim 5, wherein the shaft (110) of the needle has an outer diameter, wherein the end surfaces of the fibers (130, 230, 240, 330, 340) are arranged at a distance to each other, and wherein the distance between the end surfaces is greater than the outer diameter of the shaft.

7. The needle of claim 5, further comprising a third fiber (250), the third fiber being capable of transmitting light, wherein an end surface of the third fiber (250) is located at the bottom (224) of the bevel (120, 220, 320) in the vicinity of the end surface of the other one of the two fibers (240, 340).

8. The needle of claim 1, wherein proximal end section of the fiber (130, 230, 240, 250, 330, 340) is located in a connector part (470) for connection with a fiber cable (490) located between the needle (100, 300, 400) and a console including a light source and a light detector.

9. A system for optical tissue inspection, the system comprising

- a needle (100, 300, 400) according to claim 1,
- a light source (332) connected with one of the fibers (130, 230, 330) of the needle,
- a light detector (342) connected with another one of the fibers (240, 340) of the needle, wherein light coming from the light source and being emitted from the end surface of the one of the fibers can be detected by the light detector when entering the other one of the fibers,
- a processing unit (370) for processing the data from the light detector, and
- a monitor for visualization of the processed data.

10. The system of claim 9, further comprising

- a fiber cable (490) coupling the proximal end of a fiber (130, 230, 240, 250, 330, 340) of the needle (100, 300, 400) with the light source (332) or light detector (342).

11. A method for producing a needle according to claim 1, the method comprising the steps of:

- manufacturing the tip part including forming at least one through bore in axial direction,
- positioning and fixing an end section of at least one fiber in a respective through bore,
- connecting distal ends of the inner tube and the outer tube with the tip part, such that the at least one fiber is located in a space formed between the inner and outer tubes,
- passing the at least one fiber through an opening of the holder part, and
- connecting proximal ends of the inner tube and the outer tube with the holder part.

12. The method of claim 11, further comprising the step of polishing the surface of the bevel and the end surface of the at least one fiber such that the end surface of the at least one fiber is flush with the surface of the bevel.

13. The method of claim 11, wherein the fixing of the at least one fiber in the respective through bore is provided by gluing.

14. The method of claim 11, wherein the connecting of the inner and outer tubes with at least one of the tip part and the holder part, is provided by welding or by gluing.

15. The method of claim 11, further comprising the step of fixing a proximal end of the at least one fiber in a connector part located at the holder part.

**Patentansprüche**

1. Nadel (100, 300, 400), die Folgendes umfasst:

   - einen Spitzenteil (200, 310) mit einer Durchgangsbohrung in axialer Richtung,
   - einen Halterteil (160, 360, 460) mit einer Öffnung,
   - einen Schaft (110) mit einem Innenrohr (352) und einem Außenrohr (350), wobei die distalen Enden des Innen- und des Außenrohrs mit dem Spitzenteil (200, 310) verbunden sind, und wobei ein Raum (356) zwischen dem Innen- und dem Außenrohr gebildet wird,
   - eine Faser (130, 230, 240, 250, 330, 340), wobei die Faser in der Lage ist, Licht zu übertragen, wobei sich ein Endabschnitt der Faser in der Durchgangsbohrung des Spitzenteils (200, 310) befindet und wobei die Faser durch die Öffnung des Halterteils verläuft, **dadurch gekennzeichnet, dass** die proximalen Enden des Innen- und des Außenrohrs mit dem Halterteil (160, 360, 460) verbunden sind und sich die Faser in dem Raum (356) befindet, der durch das Innen- und das Außenrohr des Schafts (110) gebildet wird.

2. Nadel nach Anspruch 1, wobei der Spitzenteil (200, 310) ferner einen Schaftteil und eine abgeschrägte Kante (120, 220, 320) umfasst, die die Spitze der Nadel bildet.

3. Nadel nach Anspruch 1, wobei die Faser (130, 230, 240, 250, 330, 340) derart in der Durchgangsbohrung angeordnet ist, dass die Stirnfläche der Faser bündig mit der Fläche der abgeschrägten Kante (120, 220, 320) abschließt.

4. Nadel nach Anspruch 2, wobei sich eine Stirnflächen der Faser (130, 230, 330) an der Spitze (222) der abgeschrägten Kante (120, 220, 320) befindet.

5. Nadel nach Anspruch 1 mit zwei Fasern (130, 230, 240, 330, 340), wobei beide Fasern in der Lage sind, Licht zu übertragen, wobei sich eine Stirnfläche einer der Fasern (130, 230, 330) an der Spitze (222) der abgeschrägten Kante (120, 220, 320) und die andere der Fasern (240, 340) am unteren Ende (224) der abgeschrägten Kante befindet.

6. Nadel nach Anspruch 5, wobei der Schaft (110) der Nadel einen Außendurchmesser besitzt, wobei die Stirnflächen der Fasern (130, 230, 240, 330, 340) mit einem Abstand zueinander angeordnet sind und wobei der Abstand zwischen den Stirnflächen größer als der Außendurchmesser des Schaftes ist.

7. Nadel nach Anspruch 5, die ferner eine dritte Faser (250) umfasst, wobei die dritte Faser in der Lage ist, Licht zu übertragen, wobei sich eine Stirnfläche der dritten Faser (250) am unteren Ende (224) der abgeschrägten Kante (120, 220, 320) in der Nähe der Stirnfläche der anderen der beiden Fasern (240, 340) befindet.

8. Nadel nach Anspruch 1, wobei der proximale Endabschnitt der Faser (130, 230, 240, 250, 330, 340) in einem Konnektorteil (470) zum Anschluss mit einem Faserkabel (490) angeordnet ist, das sich zwischen der Nadel (100, 300, 400) und einer Konsole mit einer Lichtquelle und einem Lichtdetektor befindet.

9. System zur optischen Gewebeuntersuchung, wobei das System Folgendes umfasst:

   - eine Nadel (100, 300, 400) nach Anspruch 1,
   - eine Lichtquelle (332), die mit einer der Fasern (130, 230, 330) der Nadel verbunden ist,
   - einen Lichtdetektor (342), der mit einer weiteren der Fasern (240, 340) der Nadel verbunden ist, wobei von der Lichtquelle kommendes und von der Stirnfläche der einen der Fasern ausgesendetes Licht von dem Lichtdetektor detektiert werden kann, wenn es in die andere der Fasern eintritt.
   - eine Prozessoreinheit (370) zur Verarbeitung der Daten vom Lichtdetektor und
   - einen Monitor zur Visualisierung der verarbeiteten Daten.

10. System nach Anspruch 9, das ferner Folgendes umfasst:

    - ein Faserkabel (490), das das proximale Ende einer Faser (130, 230, 240, 250, 330, 340) der Nadel (100, 300, 400) mit der Lichtquelle (332) oder dem Lichtdetektor (342) verbindet.

11. Verfahren zur Erzeugung einer Nadel nach Anspruch 1, wobei das Verfahren die folgenden Schritte umfasst:

- Herstellen des Spitzenteils einschließlich der Realisierung mindestens einer Durchgangsbohrung in axialer Richtung,
- Positionieren und Fixieren eines Endabschnitts mindestens einer Faser in einer entsprechenden Durchgangsbohrung,
- Verbinden der distalen Enden des Innenrohrs und des Außenrohrs mit dem Spitzenteil, so dass sich die mindestens eine Faser in einem Raum befindet, der zwischen dem Innen- und dem Außenrohr gebildet wird,
- Durchführen der mindestens einen Faser durch eine Öffnung des Halterteils und
- Verbinden der proximalen Enden des Innenrohr und des Außenrohrs mit dem Halterteil.

12. Verfahren nach Anspruch 11, das ferner den Schritt des Polierens der Oberfläche der abgeschrägten Kante und der Stirnfläche der mindestens einen Faser umfasst, so dass die Stirnfläche der mindestens einen Faser bündig mit der Fläche der abgeschrägten Kante abschließt.

13. Verfahren nach Anspruch 11, wobei das Fixieren der mindestens einen Faser in der entsprechenden Durchgangsbohrung durch Verkleben erfolgt.

14. Verfahren nach Anspruch 11, wobei das Verbinden des Innen- und des Außenrohrs mit zumindest entweder dem Spitzenteil oder dem Halterteil durch Schweißen oder Verkleben erfolgt.

15. Verfahren nach Anspruch 11, das ferner den Schritt des Fixierens eines proximalen Endes der mindestens einen Faser in einem an dem Halterteil befindlichen Konnektorteil umfasst.

**Revendications**

1. Aiguille (100, 300, 400) comprenant :

- une partie pointe (200, 310) comprenant un alésage débouchant dans la direction axiale,
- une partie de retenue (160, 360, 460) comprenant une ouverture,
- une tige (110) comprenant un tube intérieur (352) et un tube extérieur (350), dans laquelle des extrémités distales des tubes intérieur et extérieur sont raccordées à la partie pointe (200, 310), et dans laquelle un espace (356) est formé entre le tube intérieur et le tube extérieur,
- une fibre (130, 230, 240, 250, 330, 340), la fibre étant capable de transmettre de la lumière, dans laquelle une section d'extrémité de la fibre est positionnée dans l'alésage débouchant de la partie pointe (200, 310), et dans laquelle la fibre passe à travers l'ouverture de la partie de retenue, **caractérisée en ce que** :

les extrémités proximales des tubes intérieur et extérieur sont raccordées avec la partie de retenue (160, 360, 460), et
la fibre est positionnée dans l'espace (356) formé par les tubes intérieur et extérieur de la tige (110).

2. Aiguille selon la revendication 1, dans laquelle la partie pointe (200, 310) comprend en outre une partie tige et un biseau (120, 220, 320) formant la pointe de l'aiguille.

3. Aiguille selon la revendication 1, dans laquelle la fibre (130, 230, 240, 250, 330, 340) est positionnée dans l'alésage débouchant de sorte que la surface d'extrémité de la fibre soit alignée avec la surface du biseau (120, 220, 320).

4. Aiguille selon la revendication 2, dans laquelle une surface d'extrémité de la fibre (130, 230, 330) est positionnée dans une partie supérieure (222) du biseau (120, 220, 320).

5. Aiguille selon la revendication 1, comprenant deux fibres (130, 230, 240, 330, 340), les deux fibres étant capables de transmettre de la lumière, dans laquelle une surface d'extrémité d'une des fibres (130, 230, 330) est positionnée dans une partie supérieure (222) du biseau (120, 220, 320) et l'autre des fibres (240, 340) est positionnée dans une partie inférieure (224) du biseau.

**6.** Aiguille selon la revendication 5, dans laquelle la tige (110) de l'aiguille possède un diamètre extérieur, dans laquelle les surfaces d'extrémité des fibres (130, 230, 240, 330, 340) sont agencées à une distance l'une de l'autre, et dans laquelle la distance entre les surfaces d'extrémité est supérieure au diamètre extérieur de la tige.

**7.** Aiguille selon la revendication 5, comprenant en outre une troisième fibre (250), la troisième fibre étant capable de transmettre de la lumière, dans laquelle une surface d'extrémité de la troisième fibre (250) est positionnée dans la partie inférieure (224) du biseau (120, 220, 320) dans le voisinage de la surface d'extrémité de l'autre des deux fibres (240, 340).

**8.** Aiguille selon la revendication 1, dans laquelle la section proximale d'extrémité de la fibre (130, 230, 240, 250, 330, 340) est positionnée dans une partie connecteur (470) pour la connexion à un câble à fibre (490) positionné entre l'aiguille (100, 300, 400) et une console comprenant une source lumineuse et un détecteur de lumière.

**9.** Système pour inspection optique de tissu, le système comprenant :

- une aiguille (100, 300, 400) selon la revendication 1,
- une source lumineuse (332) connectée à une première des fibres (130, 230, 330) de l'aiguille,
- un détecteur de lumière (342) connecté à une autre des fibres (240, 340) de l'aiguille, dans lequel de la lumière provenant de la source lumineuse et émise à partir de la surface d'extrémité de la première des fibres peut être détectée par le détecteur de lumière lors de l'entrée dans l'autre des fibres,
- une unité de traitement (370) pour traiter les données à partir du détecteur de lumière, et
- un moniteur pour visualiser les données traitées.

**10.** Système selon la revendication 9, comprenant en outre :

- un câble à fibre (490) couplant l'extrémité proximale d'une fibre (130, 230, 240, 250, 330, 340) de l'aiguille (100, 300, 400) à la source lumineuse (332) ou au détecteur de lumière (342).

**11.** Procédé pour produire une aiguille selon la revendication 1, le procédé comprenant les étapes de :

- fabrication de la partie pointe, comprenant la formation d'au moins un alésage débouchant dans la direction axiale,
- positionnement et fixation d'une section d'extrémité d'au moins une fibre dans un alésage débouchant respectif,
- raccordement des extrémités distales du tube intérieur et du tube extérieur à la partie pointe, de sorte que l'au moins une fibre soit positionnée dans un espace formé entre les tubes intérieur et extérieur,
- passage de l'au moins une fibre à travers une ouverture de la partie de retenue, et
- raccordement des extrémités proximales du tube intérieur et du tube extérieur à la partie de retenue.

**12.** Procédé selon la revendication 11, comprenant en outre l'étape de polissage de la surface du biseau et de la surface d'extrémité de l'au moins une fibre de sorte que la surface d'extrémité de l'au moins une fibre soit alignée avec la surface du biseau.

**13.** Procédé selon la revendication 11, dans lequel la fixation de l'au moins une fibre dans l'alésage débouchant respectif est fournie par collage.

**14.** Procédé selon la revendication 11, dans lequel le raccordement des tubes intérieur et extérieur à au moins une parmi la partie pointe et la partie de retenue est fourni par soudage ou par collage.

**15.** Procédé selon la revendication 11, comprenant en outre l'étape de fixation d'une extrémité proximale de l'au moins une fibre dans une partie connecteur positionnée dans la partie de retenue.

FIG. 1

FIG. 2

FIG. 3

FIG. 4

FIG. 5

**EP 2 358 265 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008008318 A